# EUROPEAN PATENT SPECIFICATION

(11) **EP 1 467 226 B1**
(45) Date of publication and mention of the grant of the patent: **06.08.2008**
(21) Application number: 03100959.0
(22) Date of filing: 10.04.2003
(51) Int. Cl.: G01T 1/24, A61B 6/00, G01N 23/04

(54) **Method for creating a contiguous image using multiple x-ray imagers**
Verfahren zur Erzeugung einem zusammenhängenden Bildes mit mehreren Röntgenabbildungsvorrichtungen
Procédé pour la formation un image contigu en utilisant multiple imageurs de rayons X

(43) Date of publication of application: 13.10.2004
(73) Proprietor: Agfa HealthCare NV, 2640 Mortsel (BE)
(72) Inventor: De Keyser, Paul AGFA-GEVAERT Corporate, 2640, Mortsel (BE)
(74) Representative: Theunis, Patrick

(56) References cited:
- EP-A- 0 421 869
- EP-A- 0 919 858
- US-A- 5 572 037
- US-B1- 6 403 964

## Description

### FIELD OF THE INVENTION

The present invention relates to a method for obtaining an image using direct digital radiographic imaging.
More specifically the invention is related to direct digital radiography using multiple sensors.

### BACKGROUND OF THE INVENTION

Traditionally, analog radiographic imaging, e.g. in medical applications, is performed using a combination of a phosphor layer, converting the X-rays to light, and a photographic film, e.g. 35 * 43cm for a relatively large field of view as used in chest x-rays. The light emitted by the phosphor is captured by the film which is developed to obtain a image on the film.

Direct methods using only a film without a phosphor layer exist but are characterised by a low efficiency and can not be used for medical applications due to the need of high radiation dose needed to expose the film.

Both methods have the drawback that the photographic film has to be chemically processed, leading to chemical waste products and loss of time.

The first digital X-ray systems, now known as computed radiography (CR)use a stored energy releasing phosphor sheet which is exposed to a radiation image during X-ray exposure. The stimulable phosphor stores the radiation image at exposure whereafter the stored energy image is read out using stimulating radiation scanning the phosphor plate, releasing the image-wise stored energy as light. The light is detected and a electronic image is generated by the light detector and processing electronics, whereafter it is digitised.

There is always a loss of time between the X-ray exposure and the readout of the image. Changing of the phosphor plates for the subsequent exposures is also cumbersome and requires intervention by the operator of the imaging apparatus.

Direct digital X-ray systems (DR) reading out the radiation image in real time using electronic sensors do exist. The systems can be divided in two main classes:
1. Systems using direct X ray conversion to electrons. The incident radiation reaches a photoconductor material which transforms the radiation into electron-hole pairs in response to the intensity of e.g. the X-rays. Charges generated are collected in a silicon based chip supporting the photoconductor.
   Conversion materials typically used are amorphous Selenium, Mo, lead iodide, mercury iodide, CdTe CdZnTe etc..
2. Presently most systems use indirect conversion sensors having a scintillator or phosphor layer as conversion layer converting the X-ray into visible light which is then detected with an electronic light sensor array which converts the light into electrons. Used materials in the conversion layer may be e.g. Gadolinium oxysulfide or cesium iodide.
   It is essential that the conversion layer absorbs a significant part of the X-rays to achieve efficient detection, and thus the lowest possible dose to the patient. Well-designed electronic system should typically reach double efficiency versus film-based X-ray detection.

### Detailed discussion of existing digital systems and drawbacks :

The majority of the current systems are flat panels made with amorphous Silicon technology. These are matrix arrays of a-Si addressing transistors covered with a photoconductor material in case of type 1. In case 2, these are matrix arrays of a-Si addressing transistors and a-Si photodiodes, covered with a phosphor. Size up to 43x43 cm is not a problem with these panels.
- A first disadvantage however is that pixel resolution is currently limited, as amorphous silicon, due to its physical properties, does hardly allow to fabricate pixels smaller than some 100 microns square.
- A second disadvantage lies in the fact that currently these panels are of the passive type, i.e. have no in-pixel amplification capability. This makes fast imaging (i.e. continuously producing more than 1 image per second) very cumbersome and expensive.

In principle, a detector using crystalline Si or another high-quality semiconductor material would be far superior in terms of smaller pixel size reachable, and due to the possibility of in-pixel circuitry such as amplifiers which greatly helps for higher imaging speeds. The major problem is that the (silicon) wafers used to make such detectors are limited in size. Large wafers are very costly and also the yield of the production process can be low. I.e. the percentage of good sensors out of a production run decreases very rapidly with increasing size.
- An existing solution, indeed making use of crystalline detectors such as CCDs or CMOS sensors for indirect detection, to this problem is that the light image generated by the conversion layer is reduced and imaged to the small image sensor by an optical system. This may be a lens system or an optical fibre system.
   The most important drawbacks of this method are:
- Due to the reduction of the image a relatively low number of pixels are read out for a large image, resulting in an inferior image quality, due to lower resolution.
- A significant portion of the light is lost, leading to lower detection efficiency and/or higher dose to the patient. This happens because with the current state of the technology, and with demagnifications higher than five to ten times, unless expensive cooling mechanisms are used, the electronic noise from the detector system will be so high in comparison with the electronic signal, that the intrinsic signal-to-noise ratio of the X-ray signal is significantly degraded.
- The bulky optics does not allow that the readout system is housed inside e.g. a conventional X-ray cassette. This would be desirable however, as a system which can be housed in a cassette with the form factor of a conventional X-ray cassette could then be plugged into a conventional X-ray apparatus. This would allow an easy upgrade from analog to digital imaging workflow, whereby the existing X-ray apparatus does not have to be replaced entirely.
   Standard cassettes are provided in several sizes for different applications and dimensions and specifications of cassettes are regulated by international approved standards such as ANSI Ph 1.49.

In order to avoid undesirable low number of pixels mentioned above, systems have been sought to split up the large field of view into smaller sections, each covered by a separate imager. The method for doing this should however :
- not show any visible greyscale response or differences between individual image sensors. This can usually be solved with proper calibration techniques.
- Not lose any image information in between the image sensors. This is not so easy to do, and several approaches exist to try to circumvent this problem. These will now be described.

Several prior art systems exist to make one large image using multiple image sensors:

Techniques try to minimise or even eliminate the spacing between the border pixels of the individual imagers by bringing them close together (butting).

Linear butting of sensors can be found in several documents :

In EP 262 267 an image-receiving plane is made up of a two-dimensional array of radiation detectors, each having his own image processing circuit. In between the sensors however insensitive gaps exist.

US 6 207 944 describes a sensor in which the edge pixels are larger in order to provide butting of the different sensors. However by providing a lager edge pixel generates a distortion to the image at the location of the butting lines.

Likewise in US 6 323 475 conductive tracks lead from the selected detector positions to offset readout circuit positions allowing for certain pixels to be bigger than others.

In WO 99/33 117 several imaging device tiles are very accurately mounted on a support structure.
Especially the very accurate mounting poses problems in fabrication and makes such a combination of imaging tiles costly. It is requires high-precision machining technology to cut the silicon very precisely. And high precision machinery is needed to mount the chips together. This is costly and complex.

In EPA-1 162 833 sensors are mounted to but sensitive areas. In one direction the chips overlie each other while in the other direction the chips are butted. The problem is that typically the edges of chips can not be brought close together than 50-100 microns, leaving a gap in between that is not light-sensitive and thus could miss vital (in other words: possibly lethal, e.g. a small cancer trace) information.

Also other systems make use of overlap of sensors in one directions in order to bring pixels closer together and not to loose information. Such system can also be found in EP 421 869. A very accurate overlapping of the chips provides butting of the sensitive areas.

In US 4 467 342 CCD chips for detecting radiant energy have an overlap joint without substantial phase difference occurring at the lap joint. The major difficulty is to align the sensors accurately. CCD sensors exhibit low efficiency for radiation image sensing.

Another approach is using multiple cameras providing overlapping images of a e.g. scintillator screen.

In US 4 503 460, DE 20000603 and EP 618 719 a combination of plural television pickup devices coupled to one X-ray intensifier using optics is used. Various detected regions overlap. The system has the drawback that due to the use of lenses the apparatus is large and can not be retrofitted in existing machines and x-ray cassettes. These systems have the advantage that no information is missing in between the separate images. They have however also a lower light efficiency, the cost of the multiple systems and due to their thickness usually do not fit in a conventional film-based X ray system.

US6 038 286 makes use of mirrors to divide the image towards several camera systems. This system has nearly the same drawbacks as it uses multiple cameras. The height is however somewhat less. Overlap seam problems may however occur at the mirror's edges.

WO 91/10921 describes the use of the transparency of silicon when using DRAM Chips having large spaces between cells as imaging sensors. Large spaces in between cells is however not acceptable for medical applications.

Other systems use mechanical step and repeat systems successively positioning the sensors at different positions. Mechanical systems are however slow, expensive and pose on the long term reliability problems. These systems can e.g. not be used for in vivo diagnostics.

In conclusion, the current state of the art is that imagers are either butted and in such case there is always a compromise on the image detail at the seams, and positioning is cumbersome and expensive; or a camera-like approach is used, but in such case the detector assembly rapidly becomes too thick to allow insertion into conventional analog X-ray units.

There is thus a need for a digital x-ray detection system possible combining several desired properties :
- having a relatively large sensing area with sufficient pixel density.
- having low enough noise to produce more than one image per second,
- being flat enough to allow insertion in conventional X-ray units,
- not missing or significantly compromising parts of the image due to imperfect butting of the different imagers the detector plane is composed off, and
- allowing at the same time low-cost manufacturing techniques for positioning the different imagers.

### SUMMARY OF THE INVENTION

The desirable features and avoidance of the above-mentioned drawbacks can be realised by a method having the specific features set out in claim 1.
Specific features for preferred embodiments of the invention are set out in the dependent claims.

Further advantages and embodiments of the present invention will become apparent from the following description and drawings.

### BRIEF DESCRIPTION OF THE DRAWINGS

- Fig. 1: illustrates the mounting of two overlapping image sensors .
- Fig. 2A: and 2B show the images acquired by the two overlapping sensor wafers during the exposure.
- Fig. 3: shows the resulting image from the combination of the two sub-images.
- Fig. 4: shows a view of 4 overlapping sensors.
- Fig. 5: shows two overlapping sensors having a conversion layer with bevelled edges.

### DETAILED DESCRIPTION OF THE INVENTION

While the present invention will hereinafter be described in connection with preferred embodiments thereof, it will be understood that it is not intended to limit the invention to those embodiments.

The drawbacks of the state of the art imaging methods are avoided by a method according to the present invention.

### PRINCIPLE

Use is made of the fact that for most x-ray diagnostic energies, the used semiconductor materials used in electronics, such as crystalline and amorphous Si and Ge, GaAs, Se, CdSe.., are due to their low atomic weight partially transparent to the radiation at wafer thickness used in electronics. On top of that, they will also hardly scatter this radiation and thus avoid blurring.

As an illustration regarding the transparency of the silicon sensors, e.g. at 20KeV, which is used in mammography, a 350 micron wafer attenuates the beam by approximately 50%. Still half of the radiation energy is available behind the wafer.
Also, below 100KeV, interaction of the Silicon with the radiation is mainly through absorption via the photo-electric effect, and there is almost no scattering present which could lead to inferior image quality such as blurring.

Even better characteristics can be obtained by thinning the wafers by an extra processing step applied to the back of the wafers. This provides better transmission of the X-ray to the underlying wafers which is especially desirable for lower energies.

This makes it possible to position the different imaging sensors in an overlapping configuration with only minor signal-to-noise compromise in the overlap region, and making it possible to acquire a contiguous image of large area starting from several sub-images. If e.g. 40% of the radiation would be lost in the silicon before being converted in the scintillator, it can be calculated from physics that the SNR degradation would be only 25% or less than 3 dB.

The parts of the sensor, which do not make use of the incident radiation are as low absorbent as possible to let the radiation pass through to the next underlying sensor where needed, while radiation conversion layers (either scintillator or photoconductor type) try to achieve high efficiency by absorbing and converting as much of the radiation as possible into light or electrons for the top sensor.

The advantage of using e.g. CMOS technology as basis for the sensor chips is that this technology is a mature technology well known and very controllable in the production process. Each pixel can typically be structured so as to be "active", i.e. having an amplification circuit on its own.
The sensors can be made with a minimum of insensitive inter-pixel area.
Also, in contrast with CCDs, CMOS sensors can be devised such that they are radiation-hard, i.e. their performance will not or only slightly degrade due to exposure with X-ray photons.

Alternatively other types of sensors such as CCD's, amorphous silicon sensors,... can still be used.

The method according to the invention is characterised in that the two dimensional silicon sensors are made to overlap each other relative to the direction of the incident radiation of the exposure.

The method according to the invention obtains a radiographic image by acquiring at least two radiographic sub-images using at least an upper and a lower 2-dimensional radiation sensor, overlapping relative to the direction of the radiation source.
A single radiographic exposure from a radiation source is used for obtaining the sub-images. Each of the sensors has a radiation sensitive area comprising pixels. The radiation sensitive area of the lower sensor is overlapped by the upper sensor.
After acquiring the sub-images they are combined to obtain the combined radiographic image.

### OVERLAP CONFIGURATION

It is possible to let the light sensitive area of the lower sensor overlap by the upper sensor without being overlapped by the radiation sensitive area of the upper sensor.
It is possible to exactly align the pixel grids of the sensors to obtain a combined image. This in itself would allow, by careful positioning, to fabricate imagers where the respective areas would be perfectly butted, i.e. versus the (point-like) X-ray source, the conversion layer edges projections would exactly touch each other, or at least within a tolerance smaller than e.g. a quarter of the pixel size. The acquired sub-images would exactly align along each other.
The physical overlap along the direction of the sensors themselves would need to vary dependent upon the angle at which the penetrating radiation reaches the sensors seams.
Although this is a possible embodiment of this invention, it would require relatively costly equipment for positioning the components with these tight tolerances.

A preferred embodiment of the present invention therefore uses sensors with overlap of the radiation sensitive areas, as projected downward on each other, away from the point-like X-ray source.
The radiation sensitive area is considered to be the area comprising sensor pixels covered by the conversion layer and where image detection is performed. This means that in this area both the radiation conversion and detection of the converted radiation by the sensor takes place.

Using this method it is possible to construct a contiguous image from the sensors overlapping sub-images which truly represents the X-ray image without the physical alignment problem.
And a total image is obtained which is guaranteed not to miss any geometrical details where the detectors meet.
Preferably the overlap between the radiation sensitive areas of the sensors is at least two rows but a larger number of overlap pixel rows can be provided.
The construction of the combined image is done in a processing section.

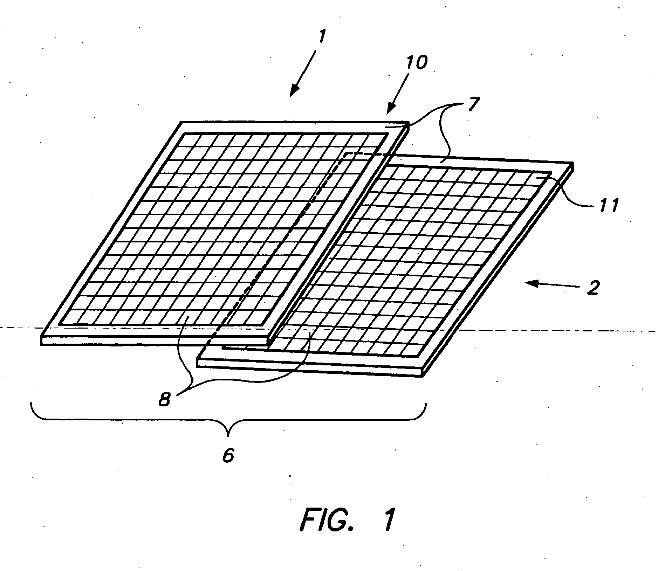

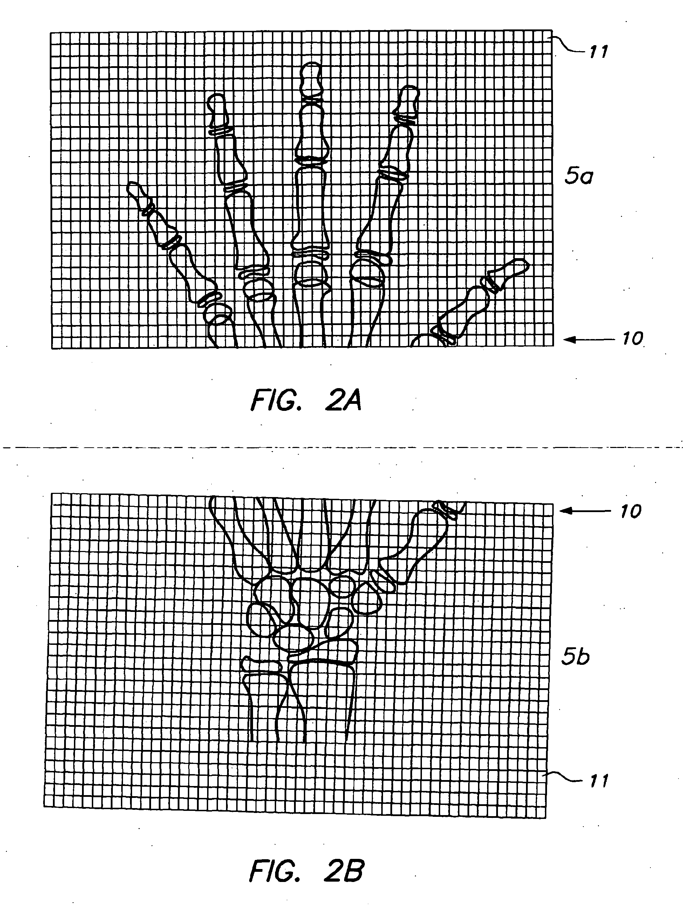

Fig 1 depicts two overlapping image sensors 1,2 as can be used in an imaging assembly 3, sensed images 5a, 5b are shown in Fig 2A and 2B. The sensors 1,2 are built on a CMOS chip comprising a non-sensitive edge 7 and a central radiation sensitive pixel grid 8 overlaid by a conversion layer 9. The conversion layer 9 can be of the direct or indirect type. As mentioned above the sensors chips 1,2 have an overlap of at least one pixel row 10 but preferably an overlap of 2 pixel rows 10 is provided.

### SINGLE EXPOSURE

The sensors 1,2 both acquire a sub-image 5a, 5b during a single radiographic exposure. This means they acquire a sub-image 5a, 5b at the same time.
A single exposure may be a continuous intensity exposure during a certain exposure time or the radiation source (e.g. X-ray tube) can be used in another mode, e.g. exposure methods are known wherein the radiation source is varied in intensity or the x-ray tube is driven in a pulsed manner. These modes are usually combined with a sensor to detect the radiation dose already received and to regulate in real-time the exposure in view the density of the object to be examined.

### SUB-IMAGE COMBINING

When combining the sub-images 5a, 5b of overlapping sensors chips 1, 2 in a processing assembly certain issues have to be addressed.

Due to variability in the fabrication of the sensor wafers 1, 2 and the application of the conversion layers 9, it is possible that not all pixel elements 11 have the same conversion efficiency.
- Calibration algorithms will also have to be used in order to correct for intensity differences in received radiation itself and in differences in gain and offset of the sensor pixels 11.
   This allows for correction of differences in conversion efficiency between sensors 1, 2 as well as between sensor pixels 11 from the same sensor wafer.
   After automatic calibration of the sub-images 5a, 5b the proper image information for each location in the image can be retrieved.
   The ultimate goal is to obtain an image wherein the signal levels or optical densities of the examined object corresponds to the physical density of the object of the radiation used.
- Another problem is a problem regarding location of the imagers 1, 2. The sub-images 5a, 5b acquired by the two overlapping sensor wafers 1, 2 in Fig. 1 during the exposure are shown in Fig. 2A and 2B.
   It can be seen that the pixel grid 8 of the sensors 1, 2 always slightly differs from the ideal position and angle. This poses problems in constructing a composite image 5 for the whole examination area.
   To avoid expensive an tedious alignment of the sensors 1, 2 the sub-images 5a, 5b are combined electronically and/or via software.
   In EP 866 342 and EP 919 858 two or more images obtained by readout of stimulable phosphor plates are likewise combined.
   Also here calibration of the algorithm is needed.
   This can be done by imaging a grid or pattern and retrieving the precise (relative) location of each sensor chip relative to the other. By interpolation algorithms and other image processing techniques, such as a two dimensional warping transformation, the sub-images 2a, 2b can be combined to one image.

As the sensors 1, 2 are mounted together in a fixed position to each other, the calibration of the algorithm has to be executed only once.
Preferably the calibration grid, which may be included in a x-ray phantom, uses a grid comprising slanted lines. This allows for detection of shift and rotation of one pixel grid 8 to another.

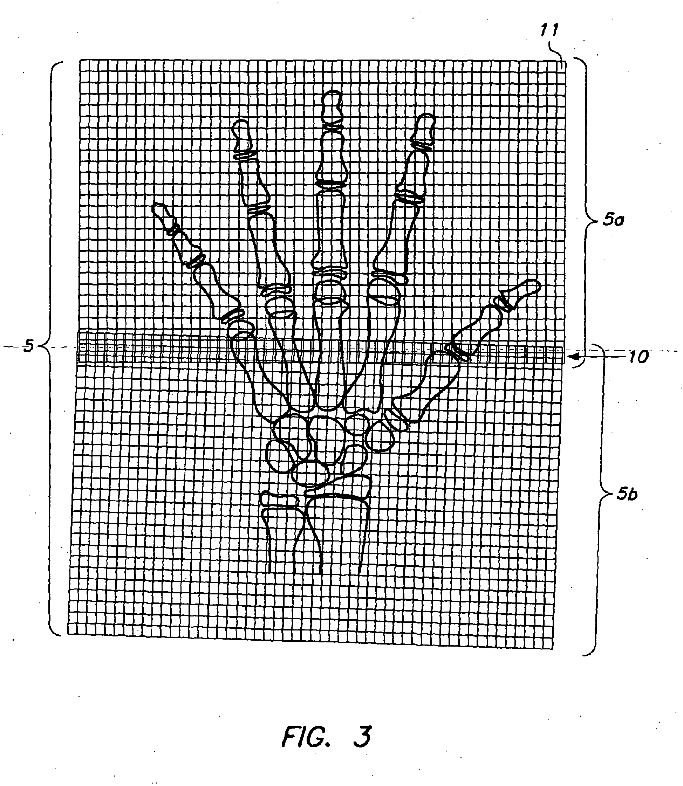

An example of the resulting image of combinations of the two sub-images 5a, 5b is shown in Fig. 3.
As mentioned the combining of the sub-images 5a, 5b is done in a processing section which is besides the radiation sensors 1, 2 also a part of the imaging assembly 6. The processing section for performing the calibrations and merging of the images 5a, 5b is normally located very close to the sensors 1, 2 but it is possible to locate the processing section at a greater distance when necessary, e.g. due to space considerations.

The advantages of a method according to the present inventions are numerous :
- A larger area can be imaged using high resolution combined imagers 1, 2, .. without inter pixel radiation insensitive areas. However, it will be clear that in case of more than 2x2 detectors, also connecting wires etc. will have to be attached such that they do not or only minimally interfere with the image.
- Easier and cheaper mounting of the sensors 1, 2, .. due to allowable overlap of the sensors 1, 2, ...
- By using overlapping sensors 1, 2, ... without optics the sensor assembly 6 can be housed in the equivalence of a state of the art X-ray cassette, giving the possibility to retrofit an existing X-ray apparatus with a module for acquiring direct X-ray images. It is also possible to retrofit a radiographic apparatus which uses stored energy releasing phosphor cassettes. At least the sensors 1, 2, ... of the radiographic imaging assembly 6 are mounted inside the radiographic film cassette. Preferably also the processing section is located inside the cassette.

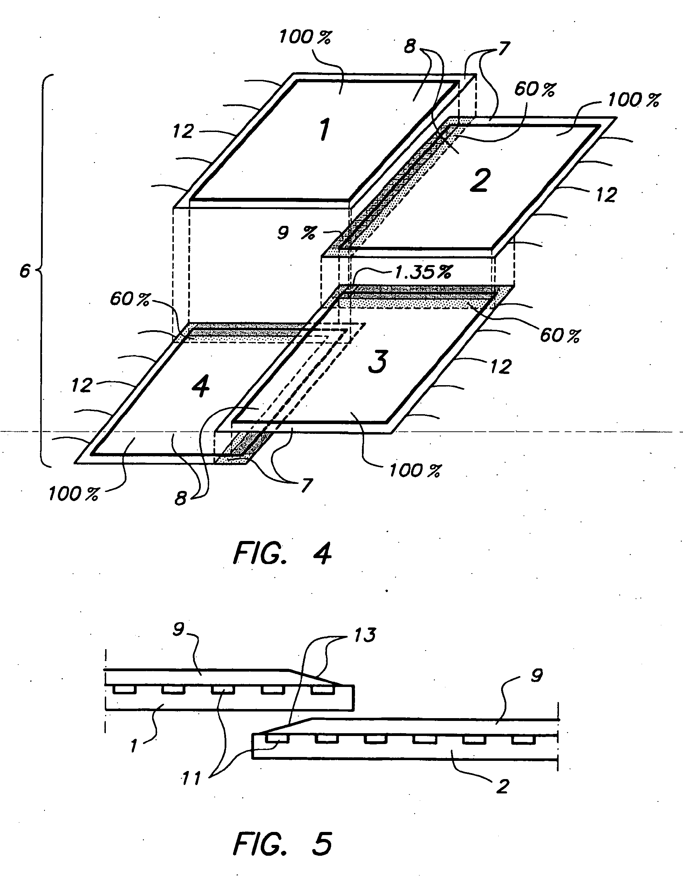

Fig. 4 gives an example of an assembly 6 of 4 overlapping sensors 1, 2, 3 and 4.
Each sensor wafer 1-4 has a dimension of about 10cm x 10cm so the combined area has a size of about 20cm x 20cm.
The wafers 1-4 are fabricated using crystalline Silicon using CMOS technology. Normally they have a thickness of about 750µm. The chips 1-4 can however be thinned by the above mentioned techniques. The thickness of the wafer 1-4 can be reduced to dimensions of 350µm.

The radiation used in this example has an energy of about 18KeV although energy levels up to 100KeV can be used. The thickness of the wafer 1 in combination with the energy of the penetrating radiation results in a approximately 50% transmission of the radiation through the wafer where it is not covered by the conversion layer 9.
The conversion layer 9 overlying the photosensitive pixel grids 8 of the wafer 1-8 is a layer of Gadolinium oxysulfide phosphor having a thickness of about 50µm. This layer has an absorption coefficient of 80% for the used radiation and thus provides a high conversion efficiency.
Preferably the sensors 1-4 are of the CMOS APS (active pixel sensor) type. These sensors utilise active transistors in each pixel 11 to buffer the photo-signal. Following characteristics can be achieved :
- X-Y addressability - small, pixel size (10um)
- Low noise ( <30e- r.m.s., single read), if need be with cooling
- High dynamic range ( 80 dB)
- Good quantum efficiency (approximately 25% peak photogate mode, 50% peak photodiode mode).
- Large formats (up to 1K x 1K demonstrated).
- Single +5 volt (or +3 volt) power supply operation.
- High Speed Electronic shutter (<100usec. exposure)
- Great Antiblooming (more than 100 x saturation)
- No image lag, >80db suppression
- On-chip timing, control, signal chain electronics
- Low Power
- Radiation resistant compared to CCDs if special measures are taken.

The voltages are readout from the chip 1-4 using the active transistors and the signals are digitised to obtain digital sub-images acquired by the separate sensors. Alternatively the sensors chips 1-4 include digitising means for direct outputting a digital image signal to the image processing means. Leads 13 to readout the sub-images from the sensors are provided at edge of the sensors 1-4 at the outside of the combined imaging region.
In the image processing sections the sub-images are combined using interpolation algorithms and other image processing techniques, such as a two dimensional warping transformation.

Possible applications of the 4 sensor assembly 6 can e.g. be found in Mammographic imaging, mobile radiology imaging, non-destructive material testing and in radiographic imaging of small mammals in veterinary radiology.

### FURTHER POSSIBLE EMBODIMENTS

The edge of the phosphor conversion layer 6 of the top wafer gives a sharp shadow in the image acquired by the underlying sensors 2-4.
In another possible embodiment shown in Fig. 5 the overlapping phosphor layers 9 can both be constructed having bevelled edges 14. This provides a smooth transition of the image of a overlying sensor 1 to the image of the underlying sensor 2. Calculations of the image information in the common area 8 can be based on the measurements of both sensors 1, 2 giving an image without any visual lines.

It can be understood that above clarification and embodiments are not restricted to X-ray apparatus. Any kind of penetrating radiation can be used, depending upon the application field and the object to be imaged.

The term X-ray sensor is therefor not limited to a sensor for so called X-ray radiation, but is to be understood as a sensor which can be used for detecting any kind of penetrating electromagnetic radiation in its broadest sense.

### SIGNAL TO NOISE CONSIDERATIONS .

Like all imaging systems the sensors exhibit a certain signal to noise ratio. The system is also a so-called photon shot noise limited system.
Each overlying sensor chip has a certain absorption and weakens the incoming signal of the underlying sensors 2-4.
For area of the bottom sensor 4 which is overlapped by 3 sensors the incoming signal is weakened and the signal-to-noise ration decreases. Let A be the adsorption percentage of the non imaging area of a single CMOS sensor chip . Then in the so-called Poisson-limited case, this means that for the fourth sensor 4 the SNR will be reduced by a factor sqrt[(1-Absorption)**3] for the areas where the penetrating radiation is unattenuated by an object.

Further noise can be added due to noise from the electronic circuits, e.g. thermal noise, before digitalisation is done.
A possible and preferable improvement of the method can be obtained by cooling the sensor chips 1-4. Thermal noise is thus reduced and its influence can be neglected. This also allows for the use of a lower radiation intensity during X-ray exposure which is important in the medical field, reducing the radiation dose administered to the patient. More specifically this will be useful in dynamic imaging procedures, where video-like sequences of images are obtained, e.g. to guide surgery. In such case, more than 1 and up to 30 images per second might be captured.
Cooling of the elements can be done using a Peltier-element or by other means available.

Normally by lowering the temperature about 8 degrees Celsius, noise can be reduced by 50%.

Most 2-dimensional radiation sensors 1-4 are of the indirect type comprising a conversion layer 9 converting the radiation into light.

A variant of the method use sensors having a direct conversion layer 9 converting the electromagnetic radiation directly to electrons.
Such layers can be made of photoconductive materials.
When the photoconductor is closely coupled to the sensing pixel a high conversion efficiency can be obtained.
However the choice of an ideal composition of the x-ray to electrons or photoconductor layer in relation to the used radiation is very important to obtain high sensitivity.
A possible direct conversion layer 9 is amorphous Selenium having a thickness of up to 500µm. To operate this sensors it is necessary to charge this layer. The photoconductor layer may for certain applications be charged with a voltage up to 5000V.

Also direct radiation sensors do exist which do not make use of conversion layers 9, but use the silicon itself instead. These sensors however require a high radiation dose and are limited to applications in the field of non destructive testing.

Having described in detail preferred embodiments of the current invention, it will now be apparent to those skilled in the art that numerous modifications can be made therein without departing from the scope of the invention as defined in the appending claims.

### [PART LIST]

- 1-4.: image sensor, sensor chip
- 5.: composite image
- 5a.: first sub-image
- 5b.: second sub-image
- 6.: imaging assembly
- 7.: non-radiation sensitive edge
- 8.: radiation sensitive grid
- 9.: conversion layer
- 10.: overlapping pixel rows
- 11.: individual pixel
- 12.: readout leads
- 13.: bevelled edges of conversion layer.

## Claims

1. Method for obtaining a radiographic image (5) comprising the steps of :
- acquiring at least two radiographic sub-images (5a,5b), during a single radiographic exposure from a radiation source, using at least an upper and a lower 2-dimensional radiation sensor (1,2,3,4) relative to the direction of the radiation source, the sensors (1,2,3,4) each having an radiation sensitive area (8) comprising pixels (11),
- combining the acquired radiographic sub-images (5a,5b), to obtain a combined radiographic image (5),
**characterised in that** the two 2-dimensional radiation sensors (1,2,3,4) overlap each other, whereby the radiation sensitive part (8) of the lower sensor is overlapped by the upper radiation sensor in the direction of the radiation source.

2. The method according to claim 1 wherein the radiation sensitive part (8) of the lower sensor is overlapped in the direction of the incident radiation of the exposure by the radiation sensitive part (8) of the upper radiation sensor.

3. The method according to claim 2 wherein the pixels (11) are arranged in rows and wherein the overlap of the radiation sensitive areas (8) is at least two rows (10).

4. The method according to any one of the claims 1 to 3 wherein the 2-dimensional radiation sensors (1,2,3,4) are indirect radiation sensors comprising a radiation to light converting layer (9).

5. The method according to any one of the claims 1 to 3 wherein the 2-dimensional radiation sensors (1,2,3,4) have a direct conversion layer (9) or photoconductor layer, which converts the electromagnetic radiation into electrons directly.

6. Method according to claim 4 or 5 wherein the conversion layer (9) is bevelled toward the edges in the overlapping region.

7. The method according any one of the claims 1 to 3 wherein the 2-dimensional radiation sensors (1,2,3,4) are direct X-ray sensors without a conversion layer (9).

8. The method according to any one of the preceding claims wherein the radiation sensors (1,2,3,4) are based on CMOS technology.

9. The method according to any one of the preceding claims wherein the radiation sensors (1,2,3,4) are cooled.

10. Radiographic imaging assembly comprising :
- at least an upper and a lower 2-dimensional radiation sensor (1,2), for acquiring at least two radiographic sub-images (5a,5b), during a single radiographic exposure
- combining means for combining the acquired images (5a, 5b) to obtain a single combined radiographic image (5), **characterized in that** the radiation sensors have overlapping radiation sensitive areas (8) in the direction of the incident radiation.

11. Radiographic imaging assembly according to claim 10 wherein the at least upper and lower radiation sensors (1,2,3,4) are mounted into a radiographic cassette for mounting into a radiographic apparatus using standard radiographic film or phosphor cassettes.

## Patentansprüche

1. Verfahren zur Erzeugung eines Röntgenbildes (5), das folgende Schritte umfasst :
- Erfassung von zumindest zwei Röntgenteilbildern (5a, 5b) während einer einzelnen Röntgenbelichtung mittels einer Strahlungsquelle, wobei relativ zur Richtung der Strahlungsquelle zumindest ein oberer und ein unterer zweidimensionaler Strahlungssensor (1, 2, 3, 4) verwendet wird und die Sensoren (1, 2, 3, 4) jeweils einen Pixel (11) enthaltenden strahlungsempfindlichen Bereich (8) umfassen,
- Kombinieren der erfassten Röntgenteilbildern (5a, 5b) zum Erhalten eines kombinierten Röntgenbildes (5),
**dadurch gekennzeichnet, dass** sich die zwei zweidimensionalen Strahlungssensoren (1, 2, 3, 4) überlappen, wobei der strahlungsempfindliche Bereich (8) des unteren Sensors durch den oberen Strahlungssensor in Richtung der Strahlungsquelle überlappt wird.

2. Verfahren nach Anspruch 1, **dadurch gekennzeichnet, dass** der strahlungsempfindliche Bereich (8) des unteren Sensors in Richtung der einfallenden Belichtungsstrahlung durch den strahlungsempfindlichen Bereich (8) des oberen Strahlungssensors überlappt wird.

3. Verfahren nach Anspruch 2, **dadurch gekennzeichnet, dass** die Pixel (11) in Reihen angeordnet sind und die Überlappung der strahlungsempfindlichen Bereiche (8) zumindest zwei Reihen (10) umfasst.

4. Verfahren nach einem der Ansprüche 1 bis 3, **dadurch gekennzeichnet, dass** die zweidimensionalen Strahlungssensoren (1, 2, 3, 4) indirekte Strahlungssensoren sind, die eine Strahlung in Licht umwandelnde Schicht (9) umfassen.

5. Verfahren nach einem der Ansprüche 1 bis 3, **dadurch gekennzeichnet, dass** die zweidimensionalen Strahlungssensoren (1, 2, 3, 4) eine direkte Umwandlungsschicht (9) oder Fotoleiterschicht, welche die elektromagnetische Strahlung direkt in Elektronen umwandelt, umfassen.

6. Verfahren nach Anspruch 4 oder 5, **dadurch gekennzeichnet, dass** die Umwandlungsschicht (9) zu den Rändern im Überlappungsbereich hin abgeschrägt ist.

7. Verfahren nach einem der Ansprüche 1 bis 3, **dadurch gekennzeichnet, dass** die zweidimensionalen Strahlungssensoren (1, 2, 3, 4) direkte Röntgenstrahlungssensoren ohne Umwandlungsschicht (9) sind.

8. Verfahren nach einem der vorstehenden Ansprüche, **dadurch gekennzeichnet, dass** die Strahlungssensoren (1, 2, 3, 4) auf CMOS-Technologie basieren.

9. Verfahren nach einem der vorstehenden Ansprüche, **dadurch gekennzeichnet, dass** die Strahlungssensoren (1, 2, 3, 4) gekühlt sind.

10. Röntgenbildgebungsanordnung, umfassend :
- zumindest einen oberen und einen unteren zweidimensionalen Strahlungssensor (1, 2) zur Erfassung von zumindest zwei Röntgenteilbildern (5a, 5b) während einer einzelnen Röntgenbelichtung, und
- Kombinierungsmittel zum Zusammenfügen der erfassten Bilder (5a, 5b) zum Erhalten eines einzelnen kombinierten Röntgenbildes (5),
**dadurch gekennzeichnet, dass** die Strahlungssensoren sich überlappende strahlungsempfindliche Bereiche (8) in Richtung der einfallenden Strahlung aufweisen.

11. Röntgenbildgebungsanordnung nach Anspruch 10, **dadurch gekennzeichnet, dass** der zumindest obere und untere Strahlungssensor (1, 2, 3, 4) in einer Röntgenkassette eingebaut sind, die wiederum in einem Standardröntgenfilm oder Leuchtkassetten nutzenden Röntgengerät verwendet wird.

## Revendications

1. Procédé pour obtenir une image radiographique (5), comprenant les étapes consistant à :
- acquérir au moins deux images secondaires radiographiques (5a, 5b), au cours d'une seule exposition radiographique à partir d'une source de rayonnement, en utilisant au moins un capteur supérieur de rayonnement bidimensionnel et au moins un capteur inférieur de rayonnement bidimensionnel (1, 2, 3, 4) par rapport à la direction de la source de rayonnement, les capteurs (1, 2, 3, 4) possédant chacun une zone sensible au rayonnement (8) comprenant des pixels (11) ;
- combiner les images secondaires radiographiques acquises (5a, 5b) pour obtenir une image radiographique combinée (5),
**caractérisé en ce que** les deux capteurs de rayonnement bidimensionnels (1, 2, 3, 4) se chevauchent mutuellement, la partie (8) sensible au rayonnement du capteur inférieur étant chevauchée par le capteur de rayonnement supérieur dans la direction de la source de rayonnement.

2. Procédé selon la revendication 1, dans lequel la partie (8) sensible au rayonnement du capteur inférieur est chevauchée, dans la direction du rayonnement incident de l'exposition, par la partie (8) sensible au rayonnement du capteur de rayonnement supérieur.

3. Procédé selon la revendication 2, dans lequel les pixels (11) sont arrangés par rangées, et dans lequel le chevauchement des zones sensibles au rayonnement (8) représente au moins deux rangées (10).

4. Procédé selon l'une quelconque des revendications 1 à 3, dans lequel les capteurs de rayonnement bidimensionnels (1, 2, 3, 4) sont des capteurs de rayonnement indirects comprenant une couche de conversion du rayonnement en lumière (9).

5. Procédé selon l'une quelconque des revendications 1 à 3, dans lequel les capteurs de rayonnement bidimensionnels (1, 2, 3, 4) possèdent une couche de conversion directe (9) ou une couche photoconductrice, qui transforme directement le rayonnement électromagnétique en électrons.

6. Procédé selon la revendication 4 ou 5, dans lequel la couche de conversion (9) est chanfreinée à proximité des bords dans la zone de chevauchement.

7. Procédé selon l'une quelconque des revendications 1 à 3, dans lequel les capteurs de rayonnement bidimensionnels (1, 2, 3, 4) sont des capteurs radiographiques directs exempts d'une couche de conversion (9).

8. Procédé selon l'une quelconque des revendications 1 à 3, dans lequel les capteurs de rayonnement bidimensionnels (1, 2, 3, 4) se basent sur la technologie CMOS.

9. Procédé selon l'une quelconque des revendications 1 à 3, dans lequel les capteurs de rayonnement bidimensionnels (1, 2, 3, 4) sont refroidis.

10. Assemblage de formation d'image radiographique, comprenant :
- au moins un capteur supérieur de rayonnement bidimensionnel et au moins un capteur inférieur de rayonnement bidimensionnel (1, 2) pour acquérir au moins deux images secondaires radiographiques (5a, 5b), au cours d'une seule exposition radiographique ;
- des moyens de combinaison pour combiner des images acquises (5a, 5b) afin d'obtenir une seule image radiographique combinée (5), **caractérisé en ce que** les capteurs de rayonnement possèdent des zones (8) sensibles au rayonnement qui se chevauchent dans la direction du rayonnement incident.

11. Assemblage de formation d'image radiographique selon la revendication 10, dans lequel ledit au moins un capteur supérieur de rayonnement et ledit au moins un capteur inférieur de rayonnement (1, 2, 3, 4) sont montés dans une cassette radiographique à monter dans un appareil radiographique dans lequel on utilise des cassettes à luminophore ou des films radiographiques standard.
